# EUROPEAN PATENT SPECIFICATION

(11) **EP 0 334 966 B1**
(45) Date of publication and mention of the grant of the patent: **24.08.1994**
(21) Application number: 88908336.6
(22) Date of filing: 16.09.1988
(51) Int. Cl.: C12P 7/62, C12P 41/00

(54) **PROCESS FOR SYNTHESIZING OPTICALLY ACTIVE COMPOUNDS**
VERFAHREN ZUR HERSTELLUNG OPTISCH AKTIVER VERBINDUNGEN
PROCEDE DE SYNTHESE DE COMPOSES OPTIQUEMENT ACTIFS

(30) Priority: 18.09.1987 JP 235300/87; 14.09.1988 JP 230773/88
(43) Date of publication of application: 04.10.1989
(73) Proprietor: NGK INSULATORS, LTD., Nagoya City Aichi Pref. (JP)
(72) Inventor: KITAZUME, Tomoya, Ohta-ku Tokyo 145 (JP)
(74) Representative: Paget, Hugh Charles Edward
(86) International application number: JP8800945
(87) International publication number: WO8902470

(56) References cited:
- JP-A- 6 221 769
- JP-A- 6 236 193
- JP-A-59 205 989
- JP-A-62 134 089
- CHEMICAL ABSTRACTS, vol. 110, no. 11, 13 March 1989, Columbus, OH (US); T. KITAZUME et al., p. 634, no. 94462s#
- CHEMICAL ABSTRACTS, vol. 109, no. 11, 1988, Columbus, OH (US); T. KITAZUME et al., no. 89261a#
- CHEMICAL ABSTRACTS, vol. 102, no. 9, 04 March 1985, Columbus, OH (US); T. KITAZUME et al., no. 75208g#
- CHEMICAL ABSTRACTS, vol. 107, no. 11, 14 September 1987, Columbus, OH (US); LIN Jenq Tain et al., no. 95238y#

## Description

### TECHNICAL FIELD

This invention relates to a method for the synthesis of optically active substances useful as ferroelectric liquid crystalline materials.

### BACKGROUND ART

Optically active substances having a halogen atom or a halogenoalkyl group on their asymmetric carbons are used as ferroelectric liquid crystalline materials. Such liquid crystalline materials have a large spontaneous polarization of not less than 150 nC/cm² and a high speed response of not more than »sec, so that they are advanced to be put into a practical use in an electro-optic effect element, a display device or the like. Recently, it has been found that such optically active substances having a fluorine atom or a fluoroalkyl group are particularly excellent liquid crystalline materials because they have an advantage that the decomposition of the liquid crystalline substance through light is less as compared with the optically active substances having another halogen atom such as chlorine, bromine or the like or other halogenoalkyl group. Their electro-optic properties are stably maintained over a long time, i.e. the degradation of the properties is very small.

The optically active substances of this type having a fluorine atom or a fluoroalkyl group are synthesized. For this purpose, an enzyme process and a non-enzyme process are adopted. The enzyme process is advantageous as compared with the non-enzyme process since both enantiomers are easily obtained.

Enzyme processes of this type are disclosed in Chemical Abstracts, Vol.102, no.9, 75208g and Vol.107, no.11, 95238y and, after the first priority date claimed in the present application, in Chemical Abstracts, Vol.109, no.11, 89621a and Vol.110, no.11, 94462s.

When the optically active substances having the fluorine atom or the fluoroalkyl group are synthesized by the enzyme process, a greater part of the steps required for the synthesis is occupied by a separation step between enzyme and unreacted substance and a separation step between enzyme and reaction product. As the separation means in such a synthesis process, chromatography is adopted. In this separation means, a long time is theoretically required in the separation and the amount of the reaction product (optically active substance) obtained by one separation operation is very small and consequently a long time is taken for obtaining several hundred grams of the optically active substance in a minimum industrial scale. Furthermore, it is necessary to repeatedly perform the combination of very complicated synthesis operation and separation operation to obtain the optically active substance at a given mixing ratio of unreacted substance to reaction product.

Honeycomb bioreactor elements are disclosed in JP-A-62-134089

### DISCLOSURE OF INVENTION

It is, therefore, an object of the invention to provide a method for synthesizing the optically active substance of this type by the enzyme process in which the separation operation at the above separation step can easily be carried out for a short time or the single separation operation itself can be omitted to reduce the number of steps for the synthesis and the mixing ratio of unreacted substance to reaction product can easily be controlled to a given value.

The invention lies in a method for the synthesis of an optically active substance as set out in claim 1

Typical and exemplary features of the invention will now be described.

The enzyme used in the invention is fundamentally a hydrolytic enzyme. This term includes various esterases as a hydrolytic enzyme for ester such as lipase and the like, and various glucosidases as a hydrolytic enzyme for glucoside such as cellulase and the like. Furthermore, the carbinol derivative used in the invention is preferably a compound having a fluorine atom or a fluoroalkyl group and represented by one of the following general formulae: 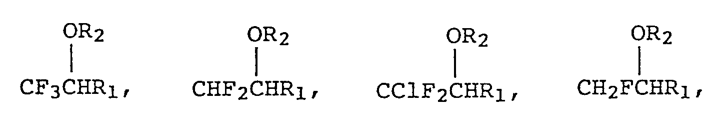
(where R₁ is a straight-chain alkyl group such as n-C₆H₁₃, n-C₈H₁₇; an ester group such as C₂H₅O(O)CCH₂; or an aromatic group such as C₆H₅CH₂CH₂; and R₂ is an acetyl group), and

R₃O(O)C-CF(R₄)-C(O)OR₃

(where R₃ is an alkyl group of 1 to 8 carbon atoms preferably a methyl or ethyl group; R₄ is a hydrogen atom, or an alkyl group of 1 to 8 carbon atoms, preferably a methyl or ethyl group). Concretely, use may be made of 2-fluoromalonic acid dimethyl ester, 2-fluoro-2-methylmalonic acid diethyl ester, acetate derivative of ethyl-4,4,4-trifluoro-3-hydroxy butanoate and acetate derivatives having a fluoroalkyl group such as CF₃, CHF₂, CF₂Cl or the like.

In the porous ceramic honeycomb structural body used in the invention, the material, average pore size, pore structure, and size and arrangement of through-holes are properly selected in accordance with the enzyme to be fixed and the conditions of the asymmetric hydrolysis reaction, and the structure body is made from a ceramic material such as cordierite, mullite, silica, alumina, zeolite, zirconia, titania or the like.

The form of through-holes in the honeycomb structural body is polygon such as triangle, square, hexagon or the like, circle, ellipsoid or the like, and its hydraulic diameter is preferably within a range of 1∼30 mm. When the hydraulic diameter of the honeycomb structural body is less than 1 mm, the fixation of the enzyme is difficult and if it is intended to pass the reaction liquid through each of the through-holes in the honeycomb structural body, the pressure loss becomes unfavorably large. On the other hand, when the hydraulic diameter exceeds 30 mm, the contact efficiency between the fixed enzyme and the reaction liquid is low and the reaction rate lowers. The open frontal area of the honeycomb structural body is preferably within a range of 50∼85%, and in this case the fixation of the enzyme is easy and the contact efficiency to the reaction liquid is high. In the honeycomb structural body after the fixation of the enzyme, even when each of the through-holes is clogged with the fixed enzyme, the use is possible, but it is preferable that a passage for flow of the reaction liquid is present in the through-hole. In the latter case, the hydraulic diameter of the through-hole is 0.5∼30 mm and the open frontal area is 30∼80%. When passing the reaction liquid through each of the through-holes within the above ranges, the pressure loss in the flow is small and the contact efficiency is high. The honeycomb structural body is preferably made from a ceramic material such as cordierite, mullite, alumina, zeolite or the like in view of the stability to a solvent in the reaction liquid. Moreover, when the ceramic material such as zeolite, γ-alumina or the like has a surface charge, ionic bonding or covalent bonding can be utilized for the fixation of the enzyme to enhance the stability of the fixed state of the enzyme. The open frontal area of the honeycomb structural body is preferably within a range of 25∼40%. When the open frontal area is less than 25%, the interaction with the enzyme is weak, while when it exceeds 40%, the strength of the honeycomb structural body lowers.

As a means for fixing the enzyme to the honeycomb structural body, use may be made of well-known processes such as inclusion process, crosslinking process, covalent bonding process, ionic bonding process, physical adsorption process and so on. Concretely, there may preferably be adopted, for example, a method wherein an enzyme-adherable substance is previously adhered to the honeycomb structural body and then the enzyme is adhered to this substance, a method wherein the enzyme is previously mixed with the above substance and then the mixture is adhered to the honeycomb structural body, a method wherein the enzyme is inserted into spaces such as pores, through-holes and the like of the honeycomb structural body and then the above substance is adhered thereto, and the like. Moreover, particularly when passing the reaction liquid through each of the through-holes in the honeycomb structural body, there may be adopted a method wherein the enzyme is carried so as to embed in the through-holes of the honeycomb structural body by the inclusion process and thereafter the excessive enzyme is removed therefrom by compressive air, vibration or the like.

In the hydrolysis reaction according to the invention, the reaction temperature is determined within a range of -20∼80°C in connection with the deactivation of enzyme. The contact time for the reaction liquid is controlled so as to obtain a given mixing ratio of unreacted substance to reaction product. Concretely, when the optically active substance is synthesized by the enzyme reaction, the optical purity generally lowers as the hydrolytic ratio becomes high, i.e. the mixing ratio of unreacted substance to reaction product approaches to 100% of reaction product, so that the contact time between the reaction liquid and the enzyme in the honeycomb structural body is determined within a range of hydrolytic ratio of 10∼80% so as to provide an optical purity of not less than 80%. In this case, the hydrolytic ratio is defined by mol ratio of (reaction product)/(unreacted substance + reaction product) in the reaction liquid × 100. When the hydrolytic ratio is less than 10%, the progress of the reaction is insufficient, while when it exceeds 80%, the optical purity largely lowers. The hydrolytic ratio is preferably within a range of 20∼70% in order to increase the yield while maintaining high optical purity. In the case of passing the reaction liquid through each of the through-holes in the honeycomb structural body, the diffusion resistance becomes small as the flow rate of the reaction liquid becomes fast, so that the flow rate is made fast to an extent of observing no peeling of the fixed enzyme. Moreover, one-pass system, circulation system and the like may properly be selected as a passing system.

In the synthesis method according to the invention, the enzyme fixed to the honeycomb structural body is used as a catalyst for obtaining the optically active substance by asymmetric hydrolysis of the carbinol derivative having a fluorine atom or a fluoroalkyl group, and the asymmetric hydrolysis reaction is carried out by contacting the enzyme in the honeycomb structural body with the carbinol derivative. Therefore, when the contact between the enzyme and the carbinol derivative is broken off after the reaction progresses to a given amount, it is sufficient to remove the honeycomb structural body from the reaction system or to remove the reaction product and the unreacted substance from the reaction system. Further, when passing the reaction liquid through each of the through-holes in the honeycomb structural body, the contact between the enzyme and the reaction liquid can be broken off without a special measure. According to the invention, therefore, the conventionally complicated separation operation requiring a long time can be made simple and short in time, or the separation operation itself can be omitted, whereby the number of steps required in the synthesis can considerably be decreased and the yield of the optically active substance can remarkably be increased. Furthermore, the progress of the asymmetric hydrolysis reaction is stopped by such a simple contact break-off method in a short time, so that the mixing ratio of unreacted substance to reaction product in the reaction system can easily be controlled to a given value, whereby the desired optically active substance can easily be obtained.

### First Example

### (1) Fixation of enzyme to honeycomb structural body

A ceramic honeycomb structural body made from mullite (outer diameter: 50 mm, length: 10 mm, through-hole form: square, pitch: 2.8 mm) was used as a honeycomb structural body, to which was fixed an enzyme by the following processes A, B and C.
- Process A:: A mixed solution of 2.5 g (13 mmol) of sodium alginate in 40 mℓ of water was adhered to the inside and outside of the honeycomb structural body, which was immersed in a 10% aqueous solution of CaCl₂ containing 7.0 g (3×10⁴ unit/g) of lipase MY *¹ to fix the enzyme thereto.
- Process B:: A mixture of 2.5 g (13 mmol) of sodium alginate, 7.5 g (3×10⁴ unit/g) of lipase MY and 40 mℓ of water was adhered to the inside and outside of the honeycomb structural body, which was immersed in a 10% aqueous solution of CaCl₂ to fix the enzyme thereto.
- Process C:: A half volume of a mixed solution of 2.5 g (13 mmol) of sodium alginate and 40 mℓ of water was adhered to a whole area of an opening portion at an end of the honeycomb structural body, while 7.0 g (3×10⁴ unit/g) of lipase MY was poured into each of through-holes from the opening portion at the other end of the honeycomb structural body to carry thereon, and then the remaining volume of the above mixed solution was adhered to a whole area of the opening portion at the other end of the structural body, which was immersed in a 10% aqueous solution of CaCl₂ to seal and fix the lipase MY.

(note)
*1: trade name of an enzyme made by The Meito Sangyo Co., Ltd.

Moreover, lipase p *², cellulase *³, or lipase M10 *⁴ was used instead of lipase MY as an enzyme and fixed in the same amount (unit/g) as in the lipase MY in these processes.
*2∼*4: trade name of an enzyme made by Amano Seiyaku Kabushiki Kaisha, respectively.

### (2) Synthesis Example I

The honeycomb structural body containing lipase MY fixed by the process A was immersed in 60 mℓ of KH₂PO₄-Na₂HPO₄ buffer solution (pH=7.3) and 20 mmol of 2-fluoromalonic acid dimethyl ester was added to this solution to conduct asymmetric hydrolysis for 1 hour with stirring at 40∼41°C, and thereafter the honeycomb structural body was removed from the solution. The resulting oily substance was extracted with diethyl ether, which was distilled under a reduced pressure after the removal of solvent to obtain (-)-2-fluoromalonic acid monomethyl ester at a yield of 67%. The properties of the product were as follows:
bp: 107∼109°C/2 mmHg
[α]_{D}/MeOH (C, 1.67): +3.22, >95%ee
¹⁹FNMR (CDCl₃): δ 114.5 (d, J_{F-H}=48Hz)ppm
¹HNMR (CDCl₃): δ 3.95 (CH₃, S), 5.42 (1H, d, J=48Hz), 10.22 (1H, S)

### (3) Synthesis Example II

The honeycomb structural body containing lipase MY fixed by the process C was immersed in 60 mℓ of the same buffer solution as in Synthesis Example I, and 20 mmol of 2-fluoro-2-methylmalonic acid diethyl ester to conduct asymmetric hydrolysis for 108 hours with stirring at 40∼41°C, and thereafter the honeycomb structural body was removed from the solution. The resulting oily substance was extracted with ethyl acetate and distilled under a reduced pressure after the removal of solvent to obtain (S)-(-)-2-fluoro-2-methylmalonic acid monoethyl ester at a yield of 75%. The properties of the product were as follows:
bp: 90∼92°C/0.6 mmHg
[α]_{D}/MeOH (C, 2.81): -17.0, 86%ee
¹⁹FNMR (CDCl₃): δ 77.8 (q, J_{F-Me}=21.4Hz)ppm
¹HNMR (CDCl₃): δ 1.32 (CH₃, t, J=7.1Hz), 1.77 (CH₃, d), 4.27 (CH₂, q), 10.90 (CO₂H, S)

### (4) Synthesis Example III

Each of various enzymes was fixed to the honeycomb structural body by anyone of the processes A, B and C, which was used to conduct asymmetric hydrolysis reaction of 2-fluoro-2-methylmalonic acid diethyl ester as shown below under the same conditions as in Synthesis Example II except for the reaction time:

CH₃CF(CO₂Et)₂ → CH₃CF(CO₂Et)CO₂H

The reaction conditions are shown in the following Table 1, and the yield and properties of the resulting reaction products are shown in the following Table 2.

**Table 1**

| Run No. | Enzyme | Fixation process | Reaction time (hr) |
|---|---|---|---|
| 1 | lipase MY | A | 24 |
| 2 | cellulase | A | 24 |
| 3 | lipase MY | B | 36 |
| 4 | lipase P | B | 42 |
| 5 | lipase MY | C | 75 |

**Table 2**

| Run No. | Yield (%) | [α]_{D} (MeOH) | Optical purity (%ee) | Absolute structure |
|---|---|---|---|---|
| 1 | 43 | -18.2 | 89 | S |
| 2 | 33 | +6.30 | 29 | R |
| 3 | 71 | -17.3 | 81 | S |
| 4 | 42 | +6.32 | 30 | R |
| 5 | 75 | -17.0 | 86 | S |

### (5) Synthesis Example IV

The honeycomb structural body containing lipase MY fixed by the process B was immersed in 60 mℓ of the same buffer solution as in Synthesis Example I, and 20 mmol of an acetate substance of ethyl-4,4,4-trifluoro-3-hydroxy butanoate was added to conduct asymmetric hydrolysis for 5 hours with stirring at 40∼41°C, and thereafter the honeycomb structural body was removed from the solution and (R)-(+) derivative was separated by a column chromatography using hexane-ethyl acetate (5:1) as a solvent. Then, the recovered acetate derivative was added to 60 mℓ of the same buffer solution as mentioned above immersing the honeycomb structural body fixed with cellulase by the process B, and the same asymmetric hydrolysis and separation as mentioned above were conducted to obtain (S)-(-) derivative. The properties of both the derivatives were as follows:
(R)-(+) derivative [α]_{D} (neat): +18.7, 88%ee
(S)-(-) derivative [α]_{D} (neat): -19.6, 92%ee
¹⁹FNMR (CDCl₃): δ 2.6 (d, J=6.6Hz)ppm
¹HNMR (CDCl₃): δ 1.25 (CH₃, t, J=7.3Hz), 2.62 (CH₂, d, J=5.6Hz), 4.30 (4×H, m)

### (6) Synthesis Example V

The honeycomb structural body containing lipase MY or lipase P fixed by the process B was immersed in 60 mℓ of the same buffer solution as in Synthesis Example I, to which were added various acetate derivatives to conduct asymmetric hydrolysis reaction as shown in the following reaction formulae (a)∼(d). Moreover, the reaction conditions and the properties of the reaction product were also shown in each of the reaction formulae.

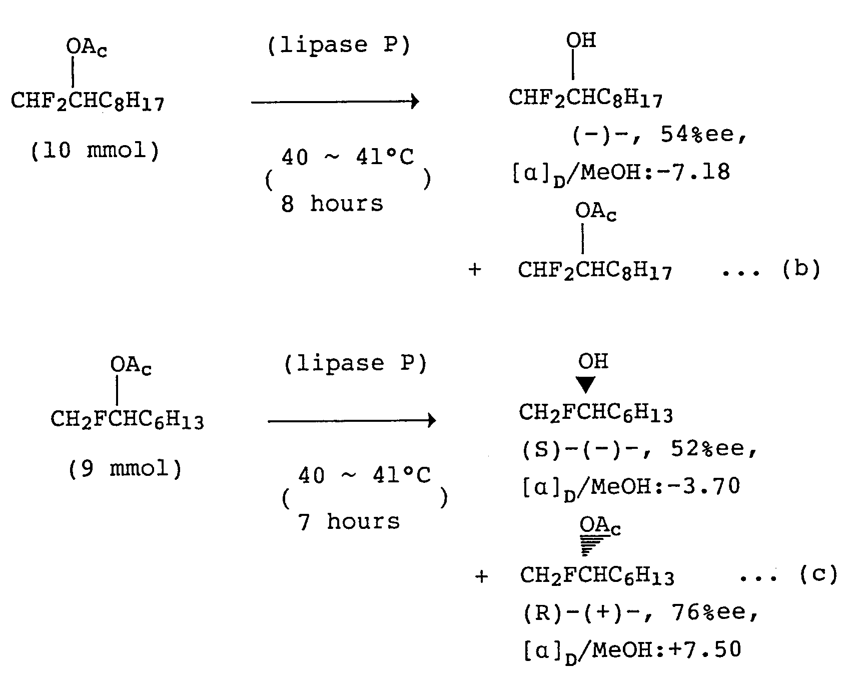

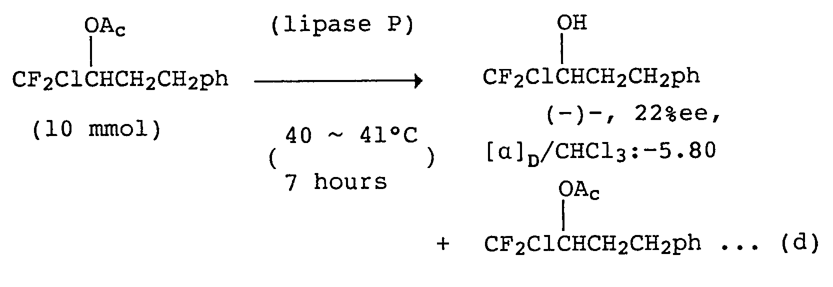

### (7) Synthesis Example VI (Comparative Example)

7.0 g (3×10⁴ unit/g) of lipase MY was dispersed into 60 mℓ of the same buffer solution as in Synthesis Example I, to which added 20 mmol of 2-fluoromalonic acid dimethyl ester, and then asymmetric hydrolysis reaction was carried out for 1 hour with stirring at 40∼41°C. After the reaction, filtration with Cellite (Regd. Trade Mark) was carried out for separating the reaction product from lipase MY, but the filtration step for 8 hours was repeated three times to take 24 hours for the separation. Then, the resulting oily substance was treated in the same manner as in Synthesis Example I to obtain (-)-2-fluoromalonic acid monomethyl ester at a yield of 74%. The properties of the product were as follows:
bp: 107∼109°C/2 mmHg
[α]_{D}/MeOH (C, 1.64): +2.95, 87%ee
¹⁹FNMR (CDCl₃): δ 114.5 (d, J=48Hz)ppm
¹HNMR (CDCl₃): δ 3.95 (CH₃, S), 5.42 (1H, d, J=48Hz), 10.22 (1H, S)

### (8) Synthesis Example VII (Comparative Example)

7.0 g (3×10⁴ unit/g) of lipase MY was dispersed into 60 mℓ of the same buffer solution as in Synthesis Example II, and 20 mmol of 2-fluoro-2-methylmalonic acid diethyl ester was added to conduct asymmetric hydrolysis reaction for 108 hours with stirring at 40∼41°C. After the reaction, the filtration with Cellite was carried out for separating the reaction product from lipase MY, but the filtration step for 8 hours was repeated three times to take 24 hours for the separation. Then, the resulting oily substance was treated in the same manner as in Synthesis Example II to obtain (S)-(-)-2-fluoro-2-methylmalonic acid monoethyl ester at a yield of 53%. The properties of the product were as follows:
bp: 90∼92°C/0.6 mmHg
[α]_{D}/MeOH (C, 2.82): -15.8, 80%ee
¹⁹FNMR (CDCl₃): δ 77.8 (q, J_{P-Me}=21.4Hz)ppm
¹HNMR (CDCl₃): δ 1.32 (CH₃, t, J=7.1Hz), 1.77 (CH₃, d), 4.27 (CH₂, q), 10.90 (CO₂H, S)

### Second Example

### (1) Fixation of enzyme to honeycomb structural body

A ceramic honeycomb structural body made from mullite (outer diameter: 50 mm, through-hole form: square, pitch: 2.8 mm, open frontal area: 75%) was used as a honeycomb structural body, to which was fixed an enzyme by the following process D.
- Process D:: A 3 wt% aqueous solution of sodium alginate and a 5 wt% suspension of lipase MY were mixed at a volume ratio of 9:1 and held at a temperature of 37°C to form a gel solution. The honeycomb structural body (length: 100 mm) was immersed into the gel solution, whereby the gel solution was adhered to the cell wall faces of the structural body. Then, compressed air was blown to adjust the thickness of the adhered film, which was immersed in a 4.5 wt% aqueous solution of CaCl₂ to conduct the fixation. After the fixation, the open frontal area was 40%.

### (2) Synthesis Example VIII

The honeycomb structural body containing lipase MY fixed by the process B was immersed in 60 mℓ of KH₂PO₄-Na₂HPO₄ buffer solution (pH=7.3), and 20 mmol of 2-fluoro-2-methylmalonic acid dimethyl ester was added to conduct asymmetric hydrolysis for 36 hours with stirring at 40∼41°C, and then the solution containing the reaction product was drawn out from the reaction system including the honeycomb structural body. The resulting oily substance was extracted with diethyl ether and then distilled under a reduced pressure after the removal of solvent to obtain (-)-2-fluoro-2-methylmalonic acid monomethyl ester having an optical purity of 84% at a yield of 70%.

### (3) Synthesis Example IX

The honeycomb structural body containing lipase MY fixed by the process D was filled in a flowing type tubular reactor, and KH₂PO₄-Na₂HPO₄ buffer solution (pH=7.3) containing 5 wt% of 2-fluoro-2-methylmalonic acid dimethyl ester was passed through each of through-holes of the honeycomb structural body to conduct asymmetric hydrolysis reaction. In this reaction system, the temperature was 40∼41°C, the flow (liquid hourly space velocity, LHSV) was LHSV=10/hr, and the contact time between the flowing liquid and enzyme in circulation system was 24 hours. The resulting oily substance was extracted with diethyl ether and then distilled under a reduced pressure after the removal of solvent to obtain (-)-2-fluoro-2-methylmalonic acid monomethyl ester having an optical purity of 85%ee at a yield of 66%.

Moreover, the reaction means of the flowing system for passing the reaction liquid through each of the through-holes in the honeycomb structural body is adopted in this synthesis example, so that the contact efficiency between reaction liquid and enzyme is good and the optically active substance having a high optical purity is obtained at a high yield, and also the separation means among the honeycomb structural body, reaction product and unreacted substance can be omitted.

According to the invention, optically active substances having very excellent properties as a ferroelectric liquid crystalline material can easily be synthesized by the contact reaction with the enzyme fixed to the porous ceramic honeycomb structural body, so that they are useful as materials for an electro-optic effect element, a display device or the like.

## Claims

1. A method for the synthesis of an optically active substance by subjecting a carbinol derivative having a fluorine atom or a fluoroalkyl group to an asymmetric hydrolysis in the presence of a hydrolytic enzyme as a catalyst, characterized in that said enzyme is fixed to a porous ceramic honeycomb structural body and is contacted with said carbinol derivative to conduct said asymmetric hydrolysis reaction, and then said enzyme in said honeycomb structural body and said carbinol derivative are separated, after said reaction has progressed to a given degree, by removing said honeycomb structural body from said reaction system or by removing reaction product and unreacted substance from said reaction system.

2. A method according to claim 1, wherein said enzyme in the honeycomb structural body and said carbinol derivative are separated to stop said asymmetric hydrolysis reaction when said asymmetric hydrolysis of said carbinol derivative has progressed to 10^{∼}80% as a hydrolytic ratio.

3. A method according to claim 1, wherein said enzyme in the honeycomb structural body and said carbinol derivative are separated by passing the reaction liquid out of through-holes in said honeycomb structural body.

4. A method according to any one of claims 1 to 3, wherein said carbinol derivative is a compound represented by one of the following general formulae: 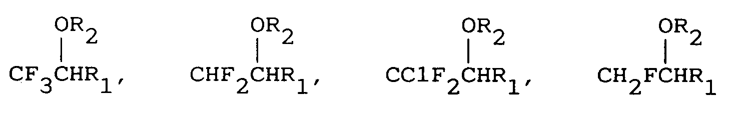 (where R₁ is a straight-chain alkyl group, an ester group or an aromatic group and R₂ is an acetyl group), and
R₃O(O)C-CF(R₄)-C(O)OR₃
(where R₃ is an alkyl group of 1 to 8 carbon atoms, and R₄ is a hydrogen atom or an alkyl group of 1 to 8 carbon atoms.

## Patentansprüche

1. Verfahren zur Synthese einer optisch aktiven Substanz, bei dem ein ein Fluoratom oder eine Fluoralkylgruppe aufweisendes Carbinolderivat einer asymmetrischen Hydrolyse in Gegenwart eines hydrolytischen Enzyms als Katalysator unterworfen wird, dadurch gekennzeichnet, daß das genannte Enzym an einem porösen Keramikkörper mit Bienenwabenstruktur fixiert ist und mit dem genannten Carbinolderivat in Kontakt gebracht wird, um die genannte asymmetrische Hydrolysereaktion durchzuführen, und das genannte Enzym im genannten Körper mit Bienenwabenstruktur und das genannte Carbinolderivat getrennt werden, nachdem die genannte Umsetzung bis zu einem bestimmten Grad fortgeschritten ist, indem der genannte Körper mit Bienenwabenstruktur aus dem genannten Reaktionssystem entfernt wird oder Reaktionsprodukt und nicht umgesetzte Substanz aus dem genannten Reaktionssystem entfernt werden.

2. Verfahren nach Anspruch 1, worin das genannte Enzym im Körper mit Bienenwabenstruktur und das genannte Carbinolderivat getrennt werden, um die genannte asymmetrische Hydrolysereaktion abzubrechen, wenn die genannte asymmetrische Hydrolyse des genannten Carbinolderivats bis 10-80% als hydrolytisches Verhältnis fortgeschritten ist.

3. Verfahren nach Anspruch 1, worin das genannte Enzym im Körper mit Bienenwabenstruktur und das genannte Carbinolderivat getrennt werden, indem die Reaktionsflüssigkeit aus Durchgangslöchern im genannten Körper mit Bienenwabenstruktur hinaus abgeleitet wird.

4. Verfahren nach einem der Ansprüche 1 bis 3, worin das genannte Carbinolderivat eine Verbindung einer der folgenden allgemeinen Formeln ist: 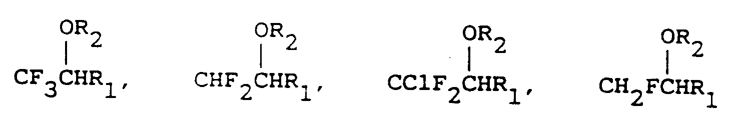 (worin R₁ eine geradkettige Alkylgruppe, eine Estergruppe oder eine aromatische Gruppe ist und R₂ eine Acetylgruppe ist) und
R₃O(O)C-CF(R₄)-C(O)OR₃
(worin R₃ eine Alkylgruppe mit 1 bis 8 C-Atomen ist und R₄ ein Wasserstoffatom oder eine Alkylgruppe mit 1 bis 8 C-Atomen ist).

## Revendications

1. Procédé pour la synthèse d'une substance optiquement active en soumettant un dérivé de carbinol ayant un atome de fluor ou un groupe fluoroalkyle à une hydrolyse asymétrique en présence d'une enzyme hydrolytique comme catalyseur, caractérisé en ce que ladite enzyme est fixée à un corps de structure en nid d'abeilles céramique poreux et est contactée par ledit dérivé de carbinol pour conduire ladite réaction d'hydrolyse asymétrique et ensuite ladite enzyme dans ledit corps de structure en nid d'abeilles et ledit dérivé de carbinol sont séparés, après que ladite réaction a atteint un degré donné, en enlevant ledit corps de structure en nid d'abeilles dudit système réactionnel ou en enlevant le produit réactionnel et la substance n'ayant pas réagi, dudit système réactionnel.

2. Procédé selon la revendication 1, où ladite enzyme dans le corps de structure en nid d'abeilles et ledit dérivé de carbinol sont séparés pour arrêter ladite réaction d'hydrolyse asymétrique quand ladite hydrolyse asymétrique dudit dérivé de carbinol a atteint 10-80% en tant que rapport hydrolytique.

3. Procédé selon la revendication 1, où ladite enzyme dans le corps de structure en nid d'abeilles et ledit dérivé de carbinol sont séparés par passage du liquide réactionnel à travers des trous dans ledit corps de structure en nid d'abeilles.

4. Procédé selon l'une quelconque des revendications 1 à 3, où ledit dérivé de carbinol est un composé représenté par l'une des formules générales suivantes : 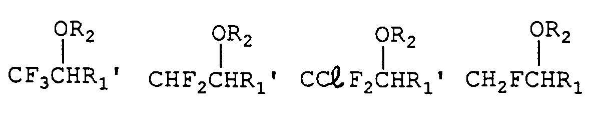 (où R₁ est un groupe alkyle à chaîne droite, un groupe ester ou un groupe aromatique et R₂ est un groupe acétyle), et
R₃O(O)C-CF(R₄)-C(O)OR₃
(où R₃ est un groupe alkyle de 1 à 8 atomes de carbone et R₄ est un atome d'hydrogène ou un groupe alkyle de 1 à 8 atomes de carbone.
